(19) Europäisches Patentamt
European Patent Office
Office européen des brevets

(11) Numéro de publication: **0 016 703**
**A1**

(12) # DEMANDE DE BREVET EUROPEEN

(21) Numéro de dépôt: 80400358.0

(22) Date de dépôt: 18.03.80

(51) Int. Cl.$^3$: C 07 D 207/327
C 07 C 101/24

(30) Priorité: 20.03.79 FR 7906946

(43) Date de publication de la demande: 01.10.80 Bulletin 80/20

(84) Etats Contractants Désignés: BE CH DE FR GB IT

(71) Demandeur: LIPHA, LYONNAISE INDUSTRIELLE PHARMACEUTIQUE
115, avenue Lacassagne B.P. No 106 Lyon R.P.
F-69212 Lyon Cedex 1(FR)

(72) Inventeur: Szarvasi, Etienne
Résidence des Grandes Bruyères
F-69260 Charbonnieres-Les-Bains(FR)

(72) Inventeur: Festal, Didier
Pins 5. Charriére Blanche
F-69130 Ecully(FR)

(74) Mandataire: Bouton Neuvy, Liliane et al,
L'Air liquide, Société Anonyme pour L'Etude et L'Exploitation des Procédés Georges Claude 75, Quai d'Orsay
F-75321 Paris Cedex 07(FR)

(54) **Sel de lysine de l'acide (+/-) amino-3 (pyrrolyl-1)-4-alpha-méthyl phényl acétique, procédé de préparation et médicament le contenant.**

(57) L'invention concerne un sel de l'acide (±) amino-3 (pyrrolyl-1)-4-α-méthyl phényl acétique. Ce sel soluble est le sel de lysine du dit acide, il est représenté par la formule

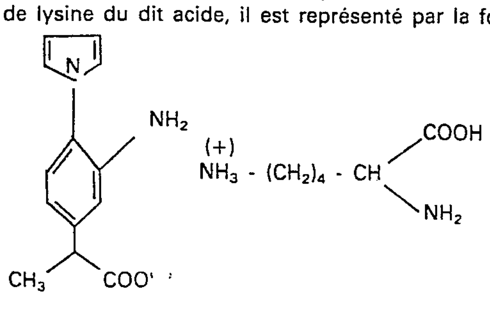

Ce nouveau composé est utilisable comme médicament analgésique et anti-inflammatoire.

EP 0 016 703 A1

"SEL DE LYSINE DE L'ACIDE (±) AMINO-3 (PYRROLYL-1)-4-∝ - METHYL PHENYL ACETIQUE, PROCEDE DE PREPARATION ET MEDICAMENT LE CONTENANT".

La présente invention concerne un sel de l'acide (±) amino-3 (pyrrolyl-1')-4 ∝ méthyl phénylacétique.

On connait les acides (±) nitro-3 et amino-3 (pyrrolyl-1')-4 ∝ - méthyl phénylacétique, respectivement dénommés composé A et composé B et représentés par la formule

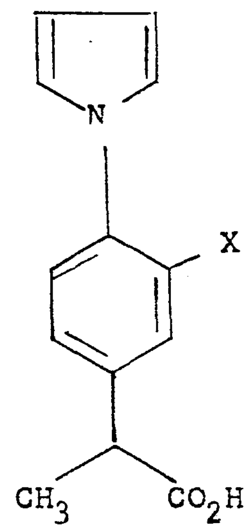

dans laquelle X est un substituant azoté choisi parmi les groupes nitro et amino, qui font l'objet du brevet d'invention France 2.371.924.

Ces composés possèdent des propriétés analgésiques et anti-inflammatoires qui les rendent utilisables comme médicaments ; toutefois ces principes actifs sont totalement insolubles dans l'eau et de ce fait ne peuvent être administrés que par la bouche.

Il a été trouvé que le sel de (±) lysine de l'acide (±) amino-3 (pyrrolyl-1')-4 ∝ - méthyl phénylacétique, dénommé composé C et représenté par la formule cidessous, possède une parfaite solubilité dans l'eau, tout en ayant de remarquables propriétés analgésique et anti-inflammatoire.

$C_{19}H_{28}N_4O_4$
P.M.=376,44

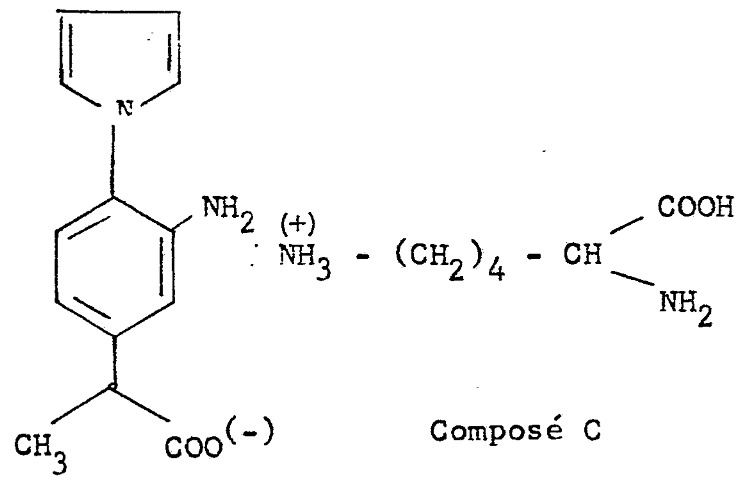

Composé C

Contrairement à la plupart des analgésiques et anti-inflammatoires commercialisés, le nouveau principe actif peut être utilisé en injections. Cette propriété du principe actif permet de traiter les douleurs et états inflammatoires intenses, qui ne répondent pas ou peu aux traitements usuels par la voie buccale.

La faible toxicité aiguë ainsi que l'activité anti-inflammatoire et analgésique du composé C ont été démontrées sur l'animal.

A - La DL 50 a été déterminée chez la souris et le rat par les voies orale et I.V., selon MILLER et TAINTER (Proc. Soc. Exp. Biol. Medecine, 1944, 57, 261.

B - L'activité analgésique a été déterminée chez la souris par la méthode de KOSTER et Col. (Fed. Proc. 1959, 18, 412). On détermine la dose efficace 50, dose qui administrée P.O. diminue de 50 % le nombre des contractions douloureuses déclenchées par l'injection intrapéritonéale d'une solution diluée d'acide acétique.

C - L'activité anti-inflammatoire a été démontrée par le test de l'oedème à la carragénine, selon WINTER et Col. (Proc. Exp. Biol. Med., 1962, 111, 544. On recherche la protection que confère le traitement par le produit administré P.O. au rat, vis à vis d'un oedème déclenché par injection sous la voûte plantaire, d'une suspension de carragénine. La dose efficace 30 est celle qui inhibe l'oedème de 30 %.

D - L'activité protectrice vis-à-vis de l'inflammation précoce a été mise en évidence sur le cobaye albinos selon la méthode de l'érythème aux U.V. (WINDER et Col., Arch. Int. Pharmacodyn. 1958, 116, 261). La dose efficace 50 est la dose qui donnée P.O., modère de 50 % l'intensité de l'érythème induit par exposition à un rayonnement U.V. de la surface dorsale épilée des cobayes.

Les résultats pharmacologiques comparés entre la butyl-4 diphényl-1,2 pyrazolidine dione-3,5 ou phénylbutazone, les composés B et C sont consignés dans le tableau suivant. La phénylbutazone sert d'étalon. Et les doses efficaces sont exprimées en mg/kg.

| | DL. 50 | | | Administration P.O | | |
|---|---|---|---|---|---|---|
| | souris P.O. | souris I.V. | rat P.O. | Acide acétique DE.50 | Oedème à la carragénine DE.30 | Erythème aux U.V. DE.50 |
| Phénylbutazone | $750 \pm 75$ | | $292 \pm 28$ | 93 | 35 | 3,7 |
| Composé B | 3000 | | $392 \pm 50$ | 19 | 12 | 4,7 |
| Composé C | $> 3200$ | $> 1600$ | $697 \pm 103$ | 42 | 15 | 4,8 |

Comme il ressort de ce tableau, le produit suivant l'invention a pratiquement la même activité que l'acide ($\pm$) amino-3 (pyrrolyl-1')-4 $\alpha$ - méthyl phényl acétique, mais sa toxicité aiguë (DL. 50) est plus favorable.

Les compositions thérapeutiques sous forme de solutions d'ampoules buvables ou injectables contenant comme principe actif, le composé du perfectionnement sont efficaces per os à des doses journalières de 200 à 600 mg et de 100 à 300 mg/kg en injection intra-musculaire.

Le sel de ($\pm$) lysine du composé B est obtenu par traitement à chaud de la lysine et du composé en solution alcoolique.

Il est donné ci-après un exemple préparatif qui illustre le perfectionnement à titre non limitatif.

EXEMPLE

A une solution de 58,12 g (0,348 mole) de (±) lysine dans 1600 $cm^3$ d'éthanol, chauffée au reflux, on ajoute 80 g (0,348 mole) d'acide (±) amino-3 (pyrrolyl-1') -4 α - méthyl phényl acétique (B).

Au bout de quelques minutes de chauffage, ce produit entre en solution. Et, après deux heures de chauffage un nouveau précipité apparait. On refroidit le mélange réactionnel entre -5 et 0° C et on essore ce précipité ; on le lave à l'éther et on le sèche sous pression réduite.

P.F. = 194 - 197° C quantité obtenue = 117,15 g

Rendement = 89 % (quantité théorique = 131g)

P.F. = 195 - 197° C ($C_2H_5OH$)

Analyse :

| | C % | H % | N % |
|---|---|---|---|
| calculé | 60,62 | 7,50 | 14,88 |
| trouvé | 60,44 | 7,47 | 14,72 |

## REVENDICATIONS

1. Sel de (±) lysine de l'acide (±) amino-3 (pyrrolyl-1')-4 α - méthyl phényl acétique, représenté par la formule.

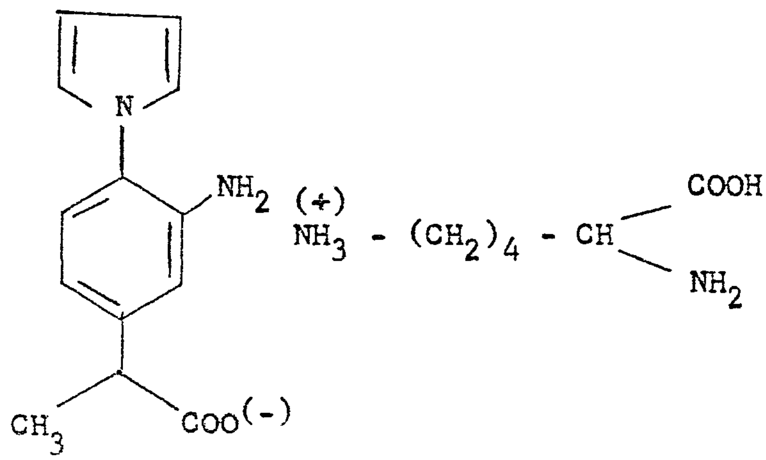

2. Procédé de préparation du sel de lysine selon la revendication 1, caractérisé en ce qu'on fait réagir à chaud en solution alcoolique la lysine et l'acide (±) amino-3 (pyrrolyl-1')-4 α - méthyl phényl acétique.

3. Médicament caractérisé en ce qu'il contient comme principe actif le sel de (±) lysine de l'acide (±) amino-3 (pyrrolyl-1')-4 α - méthyl phényl acétique selon la revendication 1.

4. Médicament selon la revendication 3, caractérisé en ce qu'il est présenté sous forme d'ampoules injectables.

5. Médicament selon la revendication 3, caractérisé en ce qu'il est présenté sous forme de solutions ou ampoules buvables.

0016703

Office européen des brevets

# RAPPORT DE RECHERCHE EUROPEENNE

Numero de la demande
EP 80 40 0358

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernee | |
| | CH - A - 488 695 (GEIGY)<br>* Page 1, formule 1, lignes 25-28 *<br>---- | 1-5 | C 07 D 207/327<br>C 07 C 101/24 |
| | | | DOMAINES TECHNIQUES RECHERCHES (Int. Cl. 3) |
| | | | C 07 D 207/327<br>C 07 C 101/24 |
| | | | CATEGORIE DES DOCUMENTS CITES<br>X: particulièrement pertinent<br>A: arrière-plan technologique<br>O: divulgation non-écrite<br>P: document intercalaire<br>T: théorie ou principe à la base de l'invention<br>E: demande faisant interference<br>D: document cité dans la demande<br>L document cite pour d'autres raisons |
| X | Le present rapport de recherche a ete etabli pour toutes les revendications | | &: membre de la même famille, document correspondant |

| Lieu de la recherche | Date d achevement de la recherche | Examinateur |
|---|---|---|
| La Haye | 17-06-1980 | MAISONNEUVE |

OEB Form 1503.1 06.78